(19) Europäisches Patentamt
European Patent Office
Office européen des brevets

(11) Veröffentlichungsnummer: **0 116 884**
**B1**

(12) # EUROPÄISCHE PATENTSCHRIFT

(45) Veröffentlichungstag der Patentschrift: **09.04.86**

(51) Int. Cl.⁴: **C 07 C 143/60**, C 07 C 143/675

(21) Anmeldenummer: **84101132.3**

(22) Anmeldetag: **04.02.84**

(54) **Nitro-aminonaphthalinsulfonsäuren.**

(30) Priorität: **16.02.83 DE 3305204**

(43) Veröffentlichungstag der Anmeldung: **29.08.84 Patentblatt 84/35**

(45) Bekanntmachung des Hinweises auf die Patenterteilung: **09.04.86 Patentblatt 86/15**

(84) Benannte Vertragsstaaten: **CH DE FR GB LI**

(56) Entgegenhaltungen:
**CH-A-532 026**
**DE-A-1 543 599**
**DE-C-216 075**
**DE-C-812 078**

(73) Patentinhaber: **BAYER AG, Konzernverwaltung RP Patentabteilung, D-5090 Leverkusen 1 Bayerwerk (DE)**

(72) Erfinder: **Hammerschmidt, Erich, Dr., Schützenstrasse 25, D-5060 Bergisch Gladbach 2 (DE)**
Erfinder: **Behre, Horst, Dr., Zur alten Linde 12, D-5068 Odenthal-Eikamp (DE)**
Erfinder: **Blank, Heinz Ulrich, Dr., Am Geusfelde 35, D-5068 Odenthal (DE)**

**EP 0 116 884 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der vorliegenden Erfindung sind Nitro-aminonaphthalinsulfonsäuren, die in Form der freien Säure der Formel

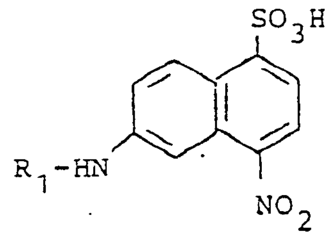

(I)

entsprechen, worin $R_1$ = H oder Acyl bedeutet.

Als Acylreste $R_1$ können beispielsweise die Reste gesättigter oder ungesättigter, offenkettiger oder cyclischer gegebenenfalls substituierter aliphatischer Carbonsäuren und Sulfonsäuren, die Reste ein- oder mehrkerniger gegebenenfalls substituierter aromatischer Carbonsäuren und Sulfonsäuren, die Reste ein- oder mehrkerniger gegebenenfalls substituierter heterocyclischer Carbonsäuren und Sulfonsäuren, heterocyclische Acylreste beispielsweise Azinylreste, wie Monazin-, Diazin- und Triazinreste, sowie die Reste der Kohlensäure und ihrer Derivate vorliegen.

Beispielsweise seien folgende Acylreste genannt:

Formyl, Acetyl, Propionyl, Butyryl, Isobutyryl, Valeryl, Isovaleryl, Pivaloyl, Hexanoyl, Heptanoyl, Octanoyl, Nonanoyl, Decancyl, Lauroyl, Myristoyl, Palmitoyl, Stearoyl, Mono-, Di- oder Trihalogenacetyl, Halogenpropionyl, Sulfonylpropionyl, Halogenacryloyl, Butadienoyl, Pyruvoyl, Acetoacetyl, 3-Acetyl-propionyl, ß-(1-Methyl)-imidazoliumpropionyl, Methacryloyl, Crotyl, Sorbinoyl, Oleoyl, Linoloyl, Methoxy- oder Aminoacetyl, Oxaloyl, Methoxalyl, Ethoxalyl, Malonoyl, Carbethoxy-acetyl, Succinoyl, Halogensuccinoyl, Glutaroyl, Adipoyl, Sebacinoyl, Maleoyl, Fumaroyl, Itaconoyl, Teraconoyl, Cyclopentancarbonyl, Cyclopentencarbonyl, Cyclopentadienoyl, Cyclohexancarbonyl, Cyclohexencarbonyl, Cyclohexadienoyl, Lactoyl, Phenylacetyl, Benziloyl, Phenylpropionyl, Cinnamoyl, Methansulfonyl, Ethansulfonyl, ß-Chlor-ethansulfonyl, Vinylsulfonyl, Benzoyl, Halogenbenzoyl, Nitrobenzoyl, niederes Alkylbenzoyl wie Toluoyl, Salicyloyl, niederes Alkylthiobenzoyl, niederes Alkoxybenzoyl wie Anisoyl, Cyanbenzoyl, Nitro-fluor-benzoyl, Nitro-chlor-benzoyl oder Nitro-brombenzoyl, Galloyl, Sulfobenzoyl, Phenylbenzoyl, Aminobenzoyl, Acylaminobenzoyl wie Formylamino-, Acetylamino-, Chlor- und Bromacetylamino-, Oxalylamino- und Benzoylaminobenzoyl, der 4-Nitro-biphenylcarbonsäure-(4)-Rest, Naphthoyl, Hydroxynaphthoyl, Anthracenoyl, Hydroxyanthracenoyl, Anthrachinoncarbonsäurereste, Benzolsulfonyl, Toluolsulfonyl, Nitrobenzolsulfonyl, Chlorbenzolsulfonyl,; ferner Furoyl, Thenoyl, Oxazol-, Thiazol-, Isothiazoloder Imidazolcarbonyl und -sulfonyl, wie 2-Chlorthia-zolcarbonyl,gegebenenfalls substituiertes niederes 2-Alkylsulfonylthiazolcarbonyl, 2-Fluorthiazolcarbonyl, 3,5-Dichlorisothiazolcarbonyl, Benzthiazolcarbonyl z.B. 2-Fluorbenzthiazolcarbonyl, 2-Chlorbenzthiazolcarbonyl oder 2-Alkyl- oder Arylsulfonylbenzthiazolcarbonyl,; Fyridincarbonyl wie Nicotinoyl, 2,6-Bis-methylsulfonyl-pyridin-4-carbonyl oder 2-Chlorpyridin-5-carbonyl, Chinolincarbonyl, Isochinolincarbonyl; pyrimidincarbonyl wie 2,4-Dichlorpyrimidin-5- oder -6-carbonyl; Phthalazincarbonyl beziehungsweise -sulfonyl, Chinoxalincarbonyl beziehungsweise -sulfonyl wie 2,3-Dichlorchinoxa-lin-6-carbonyl.

Im einzelnen seien weiterhin beispielsweise genannt.:

Mono- oder Dihalogen-sym.-triazinylreste, z.B. 2,4-Di-chlortriazinyl-6-, 2-Amino-4-chlortriazinyl-6-, 2-Alkylamino-4-chlortriazinyl-6-, wie 2-Methylamino-chlor-triazinyl-6-, 2-Ethylamino- oder 2-propylamino-4-chlor-triazinyl-6-, 2-ß-Oxethylamino-4-chlortriazinyl-6-, 2-Di-ß-oxethylamino-4-chlortriazinyl-6- und die entsprechenden Schwefelsäurehalbester, 2-Diethylamino-4-chlortriazinyl-6-, 2-Morpholino- oder 2-piperidino-4-chlortriazinyl-6-, 2-Cyclohexylamino-4-chlortriazinyl-6-, 2-Arylamino- und subst. Arylamino-4-chlortriazinyl-6-, wie 2-Phenylamino-4-chlortriazinyl-6-, 2-(o-, moder p-Carboxy- oder Sulfophenyl)-amino-4-chlortriazinyl-6-, 2-Alkoxy-4-chlortriazinyl-6-, wie 2-Methoxy- oder -Ethoxy-4-chlortriazinyl-6-, 2-(phenylsulfonylmethoxy)-4-chlortriazinyl-6-, 2-Aryloxy- und subst. Aryloxy-4-chlortriazinyl-6-, wie 2-Phenoxy-4-chlortriazinyl-6-, 2-(p-Sulfophenyl)-oxy-4-chlortriazinyl-6-, 2-(o-, moder p-Methyl- oder Methoxyphenyl)-oxy-4-chlortria-zinyl-6-, 2-Alkylmercapto- oder 2-Arylmercapto- oder 2-(subst. Aryl)-mercapto-4-chlortriazinyl-6-, wie 2-(ß-Hydroxyethyl)-mercapto-4-chlortriazinyl-6-, 2-Phenylmercapto-4-chlortriazinyl-6-, 2-(4'-Methylphe-nyl)-mercapto-4-chlortriazinyl-6-, 2-(2',4'-Dinitro)-phenylmercapto-4-chlortriazinyl-6-, 2-Methyl-4-chlor-triazinyl-6-, 2-Phenyl-4-chlor-triazinyl-6-, Mono-, Di- oder Trihalogenpyrimidinylreste, wie 2,4-Dichlor-pyrimidinyl-6-, 2,4,5-Trichlorpyrimidinyl-6-, 2,4-Dichlor-5-nitro- oder -5-methyl- oder -5-carboxymethyl-oder -5-carboxy- oder -5-cyano- oder -5-vinyl- oder -5-sulfo- oder -5-mono-, -di- oder -trichlormethyloder -5-carbalkoxy-pyrimidinyl-6-, 2,6-Dichlorpyrimi-din-4-carbonyl, 2,4-Dichlorpyrimidin-5-carbonyl-, 2-Chlor-4-methylpyrimidin-5-carbonyl-, 2-Methyl-4-chlor-pyrimidin-5-carbonyl-, 2-Methylthio-4-fluorpyrimidin-5-carbonyl-, 6-Methyl-2,4-dichlorpyrimidin-5-carbo-nyl-, 2,4,6-Trichlorpyrimidin-5-carbonyl, 2,4-Dichlor-pyrimidin-5-sulfonyl-, 2-Chlorchinoxalin-3-carbonyl-, 2- oder 3-Monochlorchinoxalin-6-carbonyl-, 2- oder 3-Monochlorchinoxalin-6-sulfonyl-, 2,3-Dichlorchinoxalin-6-carbonyl-, 2,3-Dichlorchinoxalin-6-sulfonyl-, 1,4-Di-chlorphthalazin-6-sulfonyl- oder -6-carbonyl-, 2,4-Di-chlorchinazolin-7- oder -6-sulfonyl- oder -carbonyl-, 2- oder 3- oder 4-(4',5'-Dichlorpyridazon-6'-yl-1')-

phenylsulfonyl- oder -carbonyl-, ß-(4',5'-Dichlorpyri-dazon-6'-yl-1')-ethylcarbonyl-, N-Methyl-N-(2,4-dichlortriazinyl-6-)-carbamyl-, N-Methyl-N-(2-methylamino-4-chlortriazinyl-6)-carbamyl-, N-Methyl-N-(2-dimethylamino-4-chlortriazinyl-6)-carbamyl-, N-Methyl- oder N-Ethyl-N-(2,4-dichlortriazinyl-6)-aminoacetyl-, N-Methyl-N-(2,3-dichlorchinoxalin-6-sulfonyl)-aminoace-tyl-, N-Methyl-N-(2,3-dichlorchinoxalin-6-carbonyl)-aminoacetyl-, sowie die entsprechenden Brom- und Fluor-Derivate der oben erwähnten chlorsubstituierten heterocyclischen Reste, unter diesen beispielsweise 2-Fluor-4-pyrimidinyl-, 2,6-Difluor-4-pyrimidinyl-, 2,6-Di-fluor-5-chlor-4-pyrimidinyl-, 2-Fluor-5,6-dichlor-4-pyrimidinyl-, 2,6-Difluor-5-methyl-4-pyrimidinyl-, 2,5-Difluor-6-methyl-4-pyrimidinyl-, 2-Fluor-5-methyl-6-chlor-4-pyrimidinyl, 2-Fluor-5-nitro-6-chlor-4-pyrimi-dinyl, 5-Brom-2-fluor-4-pyrimidinyl, 2-Fluor-5-cyan-4-pyrimidinyl, 2-Fluor-5-methyl-4-pyrimidinyl, 2,5,6-Trifluor-4-pyrimidinyl, 5-Chlor-6-chlormethyl-2-fluor-4-pyrimidinyl, 2,6-Difluor-5-brom-4-pyrimidinyl, 2-Fluor-5-brom-6-methyl-4-pyrimidinyl, 2-Fluor-5-brom-6-chlormethyl-4-pyrimidinyl, 2,6-Difluor-5-chlormethyl-4-pyrimidinyl, 2,6-difluor-5-nitro-4-pyrimidinyl- 2-Fluor-6-methyl-4-pyrimidinyl, 2-Fluor-5-chlor-6-methyl-4-pyrimidinyl, 2-Fluor-5-chlor-4-pyrimidiyl; 2-Fluor-6-chlor-4-pyrimidinyl, 6-Trifluormethyl-5-chlor-2-fluor-4-pyrimidinyl, 6-Trifluormethyl-2-fluor-4-pyrimidinyl, 2-Fluor-5-nitro-4-pyrimidinyl, 2-Fluor-5-tri-fluormethyl-4-pyrimidinyl, 2-Fluor-5-phenyl- oder 5-methylsulfonyl-4-pyrimidinyl, 2-Fluor-5-carbonamido-4-pyrimidinyl, 2-Fluor-5-carbomethoxy-4-pyrimidinyl, 2-Fluor-5-brom-6-trifluormethyl-4-pyrimidinyl, 2-Fluor-6-carbonamido-4-pyrimidinyl, 2-Fluor-6-carbomethoxy-4-pyrimidinyl, 2-Fluor-6-phenyl-4-pyrimidinyl, 2-Fluor-6-cyan-4-pyrimidinyl, 2,6-Difluor-5-methylsulfonyl-4-pyrimidinyl, 2-Fluor-5-sulfonamido-4-pyrimidinyl, 2-Fluor-5-chlor-6-carbomethoxy-4-pyrimidinyl, 2,6-Difluor-5-trifluormethyl-4-pyrimidinyl; sulfonylgruppenhaltige Triazinreste, wie 2,4-Bis-(phenylsulfonyl)-triazinyl-6-, 2-(3'-Carboxyphenyl)-sulfonyl-4-chlortriazinyl-6-, 2-(3'-Sulfophenyl)-sulfonyl-4-chlortriazinyl-6-, 2,4-Bis-(3'-carboxyphenylsulfonyl-1')-triazinyl-6-; sulfonylgruppenhaltige Pyrimidinringe, wie 2-Carboxymethylsulfonylpyrimidinyl-4, 2-Methylsulfonyl-6-methylpri-midinyl-4-, 2-Methylsulfonyl-6-ethyl-pyrimidinyl-4, 2-Phenylsulfonyl-5-chlor-6-methyl-p-yrimidinyl-4, 2,6-Bis-methylsulfonyl-pyrimidinyl-4, 2,6-Bis-methylsul-fonyl-5-chlor-pyrimidinyl-4, 2,4-Bis-methylsulfonyl-pyrimidin-5-sulfonyl, 2-Methylsulfonyl-pyrimidinyl-4, 2-Phenylsulfonyl-pyrimidinyl-4, 2-Trichlormethylsul-fonyl-6-methyl-pyrimidinyl-4, 2-Methylsulfonyl-5-chlor-8-methyl-Pyrimidinyl-4, 2-Methylsulfonyl-5-brom-6-methyl-pyrimidinyl-4, 2-Methylsulfonyl-5-chlor-6-ethyl-pyrimidinyl-4, 2-Methylsulfonyl-5-chlor-6-chlormethyl-pyrimidinyl-4, 2-Methylsulfonyl-4-chlor-6-methylpryimidin:S-sulfonyl,-2-Methylsulfonyl-5-nitro-6-methyl-pyrimidinyl-4, 2,5,6-Tris-methyl-sul-fonylpyrimidinyl-4, 2-Methylsulfonyl-5,6-dimethyl-py-rimidinyl-4, 2-Ethylsulfonyl-5-chlor-6-methyl-pyrimi-dinyl-4, 2-Methylsulfonyl-6-chlor-pyrimidinyl-4, 2,6-Bis-Methyl-sulfonyl-5-chlor-pyrimidinyl-4, 2-Methylsul-fonyl-6-carboxy-pyrimidinyl-4, 2-Methylsulfonyl-5-sul-fo-pyrimidinyl-4, 2-Methylsulfonyl-6-carbomethoxy-pyrimi-dinyl-4, 2-Methylsulfonyl-5-carboxy-pyrimidinyl-4, 2-Methylsulfonyl-5-cyano-6-methoxy-pyrimidinyl-4, 2-Methylsulfonyl-5-chlor-pyrimidinyl-4, 2-Sulfo-ethylsulfonyl-6-methyl-pyrimidinyl-4, 2-Methylsul-fonyl-5-brom-pyrimidinyl-4, 2-Phenyl-sulfonyl-5-chlor-pyrimidinyl-4, 2-Carboxymethylsulfonyl-5-chlor-6-methyl-pyrimidinyl-4, 2-Methylsulfonyl-6-chlor-pyrimidin-4- und -5-carbonyl-, 2,6-Bis-(methyl-sulfonyl)-pyrimidin-4- oder -5-carbonyl-, 2-Ethyl-sulfonyl-6-chlorpyrimidin-5-carbonyl-, 2,4-Bis-(methylsulfonyl)-pyrimidin-5-sulfonyl-, 2-Methylsul-fonyl-4-chlor-6-methylpyrimidin-5-sulfonyl- oder -carbonyl-; ammoniumgruppenhaltige Triazinringe, wie 2-Tri-methylammonium-4-phenylamino- oder -4-(o-, m- oder p-sulfophenyl)-aminotriazinyl-6-, 2-(1,1-Dimethylhydrazinium)-4-phenylaminooder -4-(o-, m- oder p-sulfophe-nyl)-aminotriazinyl-6-, 2-(2-Isopropyliden-1,1-di-methyl)-hydrazinium-4-phenylamino- oder -4-(o-, moder p-sulfophenyl)-aminotriazinyl-6-, 2-N-Amino-pyrrolidinium- oder 2-N-Aminopiperidinium-4-phenyl-amino- oder -4-(o-, m- oder p-sulfonphenyl)-aminotri-azinyl-6-, ferner 4-Phenylamino- oder 4-(Sulfophenyl-amino)-triazinyl-6-Reste, die in 2-Stellung über eine Stickstoffbindung das 1,4-Bis-azabicyclo-[2,2,2]-octan oder das 1,2-Bis-aza-bicyclo-[0,3,3]-octan quartär gebunden enthalten, 2-Fyridinium-4-phenylamino-oder -4-(o-, m- oder p-sulfophenyl)-amino-triazinyl-6-sowie entsprechende 2-Oniumtriazinyl-6-Reste, die in 4-Stellung durch Alkylamino-, wie Methylamino-, Ethylamino- oder ß-Hydroxyethylamino-, oder Alkoxy-, wie Methoxy- oder Alkoxy-, oder Aroxy-, wie Phenoxy- oder Sulfophenoxy-Gruppen substituiert sind; 2-Chlorbenz-thiazol-5- oder -6-carbonyl- oder -5- oder -6-sulfonyl-, 2-Arylsulfonyl- oder -Alkylsulfonylbenzthiazol-5- oder -6-carbonyl- oder -5- oder -6-sulfonyl-, wie 2-Methyl-sulfonyl- oder 2-Ethylsulfonyl-benzthiazol-5- oder -6-sulfonyl- oder -carbonyl-, 2-Phenylsulfonyl-benzthia-zol-5- oder -6-sulfonyl- oder -carbonyl- und die entsprechenden im ankondensierten Benzolring Sulfogruppen enthaltenden 2-Sulfonylbenzthiazol-5- oder -6-carbonyl-oder -sulfonyl-Derivate, 2-Chlorbenzoxazol-5- oder -6-carbonyl- oder -sulfonyl-, 2-Chlorbenzimidazol-5- oder -6-carbonyl- oder -sulfonyl-, 2-Chlor-1-methylbenzimi-dazol-5- oder -6-carbonyl- oder -sulfonyl-, 2-Chlor-4-methylthiazol-(1,3)-5-carbonyl- oder -4- oder -5-sul-fonyl-, N-Oxyd des 4-Chlor- oder 4-Nitrochinolin-5-carbonyl.

Geeignete Kohlensäure- und Kohlensäurederivate-Reste, die als Acylreste $R_1$ vorliegen können, sind beispielsweise solche der Formeln

$-CO-OR_2$ und

(II)

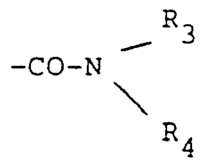

(III)

worin $R_2$ einen gegebenenfalls substituierten niederen Alkylrest oder einen gegebenenfalls substituierten Aryloder Aralkylrest darstellt und $R_3$ und $R_4$ unabhängig voneinander Wasserstoff, gegebenenfalls substituierte niedere Alkylreste oder gegebenenfalls substituierte Aryloder Aralkylreste bedeuten. Als Substituenten des Restes $R_2$ seien beispielsweise genannt: Halogenatome, wie Fluor, Chlor oder Brom; Nitro-, Cyan- und Hydroxygruppen; Alkoxygruppen mit vorzugsweise 1 - 4 Kohlenstoffatomen sowie der Phenoxyrest. Als Substituenten der Reste $R_3$ und $R_4$ kommen beispielsweise in Betracht Alkylreste mit 1 - 4 Kohlenstoffatomen, Carbonsäure- und Sulfonsäuregruppen sowie deren Ester, niedere Alkylsulfonylgruppen, Arylsulfonylgruppen, der Trifluormethylrest sowie die als Substituenten des Restes $R_2$ angeführten Atome bzw. Reste.

Gegenstand der Erfindung ist weiterhin ein Verfahren zur Herstellung der Nitroaminonaphthalinsulfonsäuren (I), das darin besteht, daß man 4-Nitro-6-acylaminonaphtha-lin-1,5-disulfonsäuren (IV), insbesondere die 4-Nitro-6-acetylaminonaphthalin-1,5-disulfonsäure sauer hydrolysiert und gegebenenfalls anschließend acyliert.

Die Hydrolyse erfolgt vorzugsweise mit starken Säuren wie Schwefelsäure, Salzsäure oder Phosphorsäure, insbesondere mit etwa 2 %-iger bis 55 %-iger Schwefelsäure, bei Temperaturen von etwa 10 - 120°C, insbesondere 30 - 70°C.

Die Acylierung erfolgt in an sich bekannter Weise mit Reste $R_1$ einführenden Acylierungsmitteln, beispielsweise den entsprechenden Säurehalogeniden, insbesondere den Säurechloriden, oder den entsprechenden Säureanhydriden, insbesondere Acetanhydrid, oder den heterocyclischen Säurederivaten wie beispielsweise den Halogentriazinen.

Gegenstand der vorliegenden Erfindung ist auch ein Verfahren zur Herstellung von Diaminonaphthalinsulfonsäuren, die in Form der freien Säure der Formel

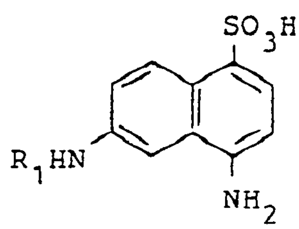

IV

entsprechen, worin $R_1$ die oben angegebene Bedeutung hat und das dadurch gekennzeichnet ist, daß man die Nitro-Verbindungen der Formel I in an sich bekannter Weise, z.B. mit katalytisch erregtem Wasserstoff, Eisen/Essigsäure, Zink/Salzsäure, Alkalisulfiden oder Alkalisulfiten reduziert.

Die Verbindungen IV sind Zwischenprodukte für die Herstellung von Azofarbstoffen. Sie können beispielsweise als Diazoverbindungen Verwendung finden oder etwa nach Austausch der 4-ständigen Aminogruppe gegen eine Hydroxygruppe nach dem in der Deutschen Patentschrift 1 543 600 beschriebenen Verfahren (Umsetzung mit wäß-riger Alkalihydrogensulfit-Lösung bei etwa 80°C bei Kochtemperatur) als Kupplungskomponente.

Die neuen Verbindungen können in Form ihrer freien Säure oder aber ihrer Salze, die nach üblichen Verfahren erhältlich sind, Verwendung finden. Bevorzugt sind dabei die Alkalisalze, insbesondere die Natriumsalze.

**Beispiel 1**

0,1 mol 4-Nitro-6-acetylaminonaphthalin-1,5-disulfon-säure wird in 800 ml $H_2SO_4$ 20 % 3 h auf 40°C erwärmt. Nach dem Abkühlen auf 20°C wird die 4-Nitro-6-amino-naphthalin-1-sulfonsäure in Form goldgelber, sandiger Kristalle isoliert. Ausbeute 96 % der Theorie.
(UV: $\lambda_{max}$ 219, 223, 347 und 410 nm)

**Beispiel 2**

27 g 4-Nitro-6-aminonaphthalin-1-sulfonsäure werden in 100 ml Wasser suspendiert und mit Natriumhydroxid auf pH 8,0 gestellt. Nach Zusatz von 20 g Acetanhydrid wird die Mischung für 15 min. auf 80°C erwärmt. Anschließend läßt man abkühlen und salzt mit Kochsalz die gebildete 4-Nitro-6-acetaminonaphthalin-1-sulfonsäure aus. Das intensiv gelb gefärbte Produkt wird mit 94 % d.Th. erhalten.

(UV: $\lambda_{max}$ 221, 243 und 345 nm)

**Beispiel 3**

0,1 mol 4-Nitro-6-acetylaminonaphthalin-1-sulfonsäure wird in 4 h in eine siedende Suspension aus 60 g Eisenspänen und 400 ml Wasser eingetragen. Dabei wird mit Schwefelsäure der pH im Reduktionskolben auf 5,0 bis 6,0 eingestellt und gehalten. Nach Beendigung des Eintragens wird noch 30 Minuten nachgerührt, dann sodaalkalisch gestellt, der Eisenschlamm abgetrennt und mit Wasser gewaschen. Das Filtrat wird mit Schwefelsäure auf pH 2 angesäuert und die ausfallende 4-Amino-6-acetylaminonaphthalin-1-sulfonsäure mit 90 % der Theorie isoliert.

(UV: $\lambda_{max}$ 223, 260 und 325 nm)

**Patentansprüche**

1. Nitroaminonaphthalinsulfonsäuren, die in Form der freien Säure der Formel

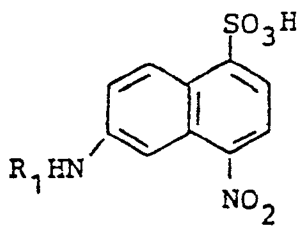

entsprechen, worin $R_1$ für Wasserstoff oder einen Acylrest steht.

2. Verbindungen des Anspruchs 1, worin $R_1$ = Rest eineraliphatischen, aromatischen oder heterocyclischen Carbonsäure oder Sulfonsäure, Rest der Kohlensäure oder ihrer Derivate oder ein heterocyclischer Acylrest ist.

3. Verbindungen des Anspruchs 1, worin $R_1$ für Acetyl, Chloracetyl, Benzoyl oder Oxaloyl steht.

4. Verfahren zur Herstellung der Verbindungen des Anspruchs 1, dadurch gekennzeichnet, daß man 4-Nitro-6-acylaminonaphthalin-1,5-disulfonsäuren sauer hydrolysiert und gegebenenfalls anschließend mit den Rest $R_1$ einführenden Acylierungsmitteln behandelt.

5. Verfahren Zur Herstellung von Diaminonaphthalinsulfonsäuren, die in Form der freien Säure der Formel

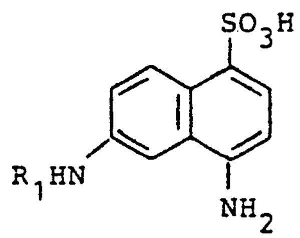

entsprechen, worin $R_1$ die in Anspruch 1 angegebene Bedeutung hat, dadurch gekennzeichnet, daß man in den Verbindungen des Anspruchs 1 die Nitrogruppe mit Wasserstoff, Eisen, Alkalisulfiden oder Alkalisulfiten reduziert.

**Claims**

1. Nitroaminonaphthalenesulphonic acids which, in the form of the free acid, correspond to the formula

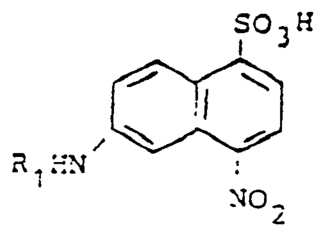

wherein

$R_1$ represents hydrogen or an acyl radical.

2. Compounds of Claim 1,

wherein

$R_1$ = a radical of an aliphatic, aromatic or heterocyclic carboxylic acid or sulphonic acid, a radical of carbonic acid or its derivatives, or a heterocyclic acyl radical.

3. Compounds of Claim 1,

wherein

$R_1$ represents acetyl, chloroacetyl, benzoyl or oxaloyl.

4. Process for preparing the compounds of Claim 1, characterised in that 4-nitro-6-acylaminonaphthalene-1,5-disulphonic acids are hydrolysed under acid conditions and then, if appropriate, treated with acylating agents which introduce the $R_1$ radical.

5. Process for preparing diaminonaphthalenesulphonic acids which, in the form of the free acid, correspond to the formula

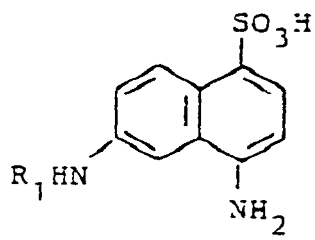

wherein

$R_1$ has the meaning given in Claim 1,

characterised in that the nitro group in the compounds of Claim 1 is reduced with hydrogen, iron, alkali metal sulphides or alkali metal sulphites.

**Revendications**

1. Acides nitro-aminonaphtalènesulfoniques qui, sous la forme de l'acide libre, répondent à la formule

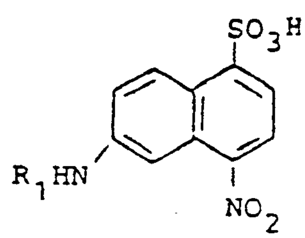

dans laquelle $R_1$ représente l'hydrogène ou un reste acyle.

2. Composés suivant la revendication 1, dans lesquels $R_1$ est le reste d'un acide carboxylique ou sulfonique aliphatique, aromatique ou hétérocyclique, le reste de l'acide carbonique ou de ses dérivés ou un reste acyle hétérocyclique.

3. Composés suivant la revendication 1, dans lesquels $R_1$ est un groupe acétyle, chloracétyle, benzoyle ou oxaloyle.

4. Procédé de production des composés suivant la revendication 1, caractérisé en ce qu'on hydrolyse en milieu acide des acides 4-nitro-6-acylaminonaphtalène-1,5-disulfoniques, puis on les traite éventuellement avec des agents d'acylation introduisant le reste $R_1$.

5. Procédé de production d'acides diaminonaphtalènesulfoniques qui, sous la forme de l'acide libre, répondent à la formule

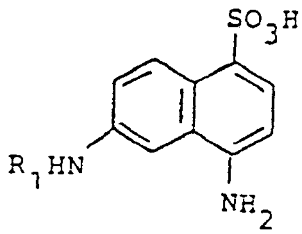

dans laquelle $R_1$ a la définition donnée dans la revendication 1, caractérisé en ce qu'on réduit dans les composés suivant la revendication 1 le groupe nitro avec de l'hydrogène, du fer, des sulfures alcalins ou des sulfites alcalins.